# EUROPÄISCHE PATENTSCHRIFT

(11) **EP 0 727 481 B1**
(45) Veröffentlichungstag und Bekanntmachung des Hinweises auf die Patenterteilung: **16.04.2003**
(21) Anmeldenummer: 96101941.1
(22) Anmeldetag: 10.02.1996
(51) Int. Cl.: C12M 3/06, C12M 1/40

(54) **Verfahren zur Kultivierung von Organfunktionszellen**
Method of cultivating functioning cells from organs
Procédé de culture des cellules des organes

(30) Priorität: 16.02.1995 DE 19505109
(43) Veröffentlichungstag der Anmeldung: 21.08.1996
(73) Patentinhaber: Forschungszentrum Jülich GmbH, 52425 Jülich (DE)
(72) Erfinder: Wandrey, Christian, Prof. Dr., D-52428 Jülich (DE); Biselli, Manfred, Dr., D-52428 Jülich (DE); Schröder, Bernd, D-52428 Jülich (DE); Schmoll, Hans-Joachim, Prof. Dr., D-30175 Hannover (DE)

(56) Entgegenhaltungen:
- EP-A- 0 113 328
- EP-A- 0 531 733
- WO-A-86/05202
- US-A- 4 564 532
- US-A- 4 681 582
- US-A- 4 978 616
- US-A- 4 987 068
- US-A- 5 242 826
- PATENT ABSTRACTS OF JAPAN vol. 007, no. 161 (C-176), 15. Juli 1983 (1983-07-15) & JP 58 071884 A (SUMITOMO BAKELITE KK), 28. April 1983 (1983-04-28)

## Beschreibung

Die Erfindung bezieht sich auf ein Verfahren zur Kulti vierung von menschlichen bzw. tierischen Organfunktionszellen, bei dem auf porösen Mikroträgern angesiedelte Zellen kontinuierlich mit Wachstumsmedium versorgt werden.

Die ex vivo Kultivierung von Säugerzellen mit Organfunktion oder -teilfunktion, wie von menschlichem Gewebe, Knochenmark, Leber, Haut etc., ist von hohem Interesse für medizinische und pharmazeutische Anwendungen sowie im Forschungs- und Entwicklungsbereich: So werden für verschiedene klinische Behandlungen Knochenmark, Nabelschnurvenenblut cder auch aus dem Blut gewonnene periphere Stammzellen benötigt.

Von besonderem Interesse ist derzeit im Bereich der Leukämie-Behandlung die Kultivierung von Knochenmark-Stammzellen im Hinblick auf eine Vermehrung oder zumindest "Gleichgewichts-Erhaltung", um so die erhebliche Spenderproblematik und Probleme der Akzeptanz bei den bislang bekannten oder in Erwägung gezogenen Methoden in den Griff zu bekommen, wonach z.B.
(1) bei der autolcgen Knochenmarktransplantation wird Knochenmark dem Empfänger selbst entnommen und z.B. nach einer Krebstherapie zurückgegeben. Als zukünftige Therapieform wird auch die Behandlung oder Heilung von leukämische oder tumorinfiltrierten Knochenmark außerhalb des Patienten in Erwägung gezogen (*ex vivo* purging).
(2) Bei allogenen Knochenmarktransplantationen wird das gesunde Knochenmark eines Spenders einem Leukämiepatienten gegeben, dem vorher eigenes Knochenmark entfernt wurde. Dabei kann als Komplikation auftreten, daß das fremde Knochenmark abgestoßen wird oder das Empfängergewebe als fremd ansieht und bekämpft wird (Graft versus host reaction).

Bis vor kurzem wurde in allen Fällen Knochenmark nur als Zellgemisch transplantiert. Seit kurzem werden die Stammzellen aus dem Knochenmark durch die Zugabe von Zytokinen in das periphere Blut herausgeschwemmt (mobilisiert) und aus dem peripheren Blut gewonnen. Diese Stammzellen werden dann immunologisch aufgereinigt und dem Patienten transplantiert. Somit wird dem Spender eine Entnahme den Knochenmarks erspart, die schmerzhaft ist und den korper auch stark belastet. Bei der peripheren Stammzellentnahme werden nur die gewünschten Stammzellen entnommen, die übrigen Zellen erhält der Spender zurück. Somit ist der Verlust an hämapoetischen Zellen beim Spender sehr gering.

Durch ein Verfahren zur Vermehrung oder doch zumindest Aufrechterhaltung der Menge solcher Organfunktionszellen würde diese neue Behandlungstechnik erheblich gefördert.

Die erhaltende oder vermehrende Kultivierung von Progenitorzellen (Knochenmark-Stammzellen) wäre darüber hinaus geeignet für die Produktion von aus eigenen (autolog) oder Spenderstammzellen (allogen) gebildeten reifen Blutzellen, die dann für Bluttransfusionen als Spendenersatz für Blutzellen, wie z.B. Erythrozyten, Thrombozyten und Granulozyten genutzt werden könnten. Dadurch würde die Ansteckungsgefahr für den Blutempfänger stark gemindert oder eliminiert und eine gleichbleibende Produktqualität gewährleistet.

Mit Hilfe der erhaltenden oder vermehrenden Kultivierung könnte schließlich eine Spendenbank angelegt werden, aus der die jeweils benötigten spezifischen stammzellen für eine Transplantation bereitgestellt werden könnten.Dies würde den derzeit enormen Aufwand und Zeitdruck mindern.

Es gibt daher bereits seit über zehn Jahren erhebliche Bemühungen, Organfunktionszellen und gewebeähnliche Mischpopulationen zu kultivieren und möglichst eine Vermehrung zu erzielen. Echte Langzeitkulturen sind jedoch immer noch außerordentlich problematisch.

Bekannt sind Versuche zur Kultivierung von Knochenmarkpräparaten, insbesondere von hämapoetischen Stammzellen, auf Stromazellen bzw. Fibroblasten, die zunächst auf einem insbesondere aus Nylon bestehenden Netzwerk angezüchtet werden, bevor mit hämapoetischen Zellen beimpft wird (EP 0241578 A1).

Dabei sollen auch im Wachstumsmedium suspendierte Träger beliebiger Konfiguration vorgesehen und mit periodischem Medienaustausch gearbeitet werden (EP 0358506).

Für die *ex vivo* Kultivierung von hämapoetischen Vorläuferzellen wird auch eine vorangehende Behandlung mit bzw. die Anwesenheit von Wachstumsfaktoren bzw. Zytokinen empfohlen (WO 92/21404).

In der WO 93/18136 wird die Kultivierung von hämapoetischen Zellen auf Mikroträger-Perlen mit kontinuierlicher oder intermittierender Wachstumsmedienzufuhr vorgeschlagen, die im Rührkessel- oder "Airlift"-Bioreaktor durchführbar sein soll. Beschrieben wird die Kultivierung in Spinner-Flaschen mit auf Kollagen aufgewachsenen Zellen.

Die internationale Patentanmeldung PCT/US86/00260 offenbart ein Verfahren und eine Vorrichtung bei dem Kulturzellen in einem nach unten chronisch zulaufenden Reaktor in einem Kreisprozeß geführt werden.

Das amerikanische Patent mit der Nummer 4,978,616 offenbart eine ähnliche Vorrichtung, bei der mehrere Reaktoreinheiten gemäß der Wo 86/05202 in Serie geschaltet sind.

Das amerikanische Patent mit der Nummer 4,987,068 offenbart ein Verfahren bei dem Mikroorganismen oder tierische Zellen in einem Bioreaktor kultiviert werden, bei dem poröse anorganische Sinterkörper mit offenen Poren verwendet werden.

Die europäische Patentanmeldung mit der Nummer 05 31 733 A1 betrifft einen Trägerkörper für tierische Zellkulturen der aus metallischem Material besteht und eine dreidimensionale Netzwerkstruktur hat. Auf dem Carrier sind zellacesive Strukturen sowie Zellwachsumsfaktoren immobilisiert.

Schließlich beschreiben T.-Y. Wang u. a. (Anm. NY. A-cad. Sci. 1990, Seiten 274-284) eine Drei-Kammer-Anordnung zur Kultivierung von hämapoetischen Zellen, die auf hochporösen Kollagen-Mikrokugeln wachsen. Diese werden als Packung in der mittleren Kammer vorgesehen und sind von den angrenzenden, von Wachstumsmedium durchströmten Kammern durch eine Perfusionsmembran getrennt.

Auch diese Technik kann offensichtlich noch nicht als echte Langzeizkultivierung angesehen werden.

Ziel der Erfindung ist daher eine Verfahrensweise, die eine Replikation von Organfunktionszellen, insbesondere von Stammzellen in dem Maße erlaubt, daß Verluste durch Ausdifferenzierung aufgewogen werden. D.h., der *ex vivo* bislang beobachtete zunehmende Schwund bis zum Absterben der Kultur soll vermieden und möglichst ein signifikanter Überschuß wiederholt geerntet werden können.

Obwohl das derzeitige Interesse vornehmlich den Knochenmark-Stammzellen gilt, weshalb sich die vorliegende Beschreibung weitgehend darauf bezieht, ist die erfindungsgemäße Technik nicht darauf beschränkt, sondern selbstverständlich mit anderen Organfunktionszellen oder Zellmischpopulationen anwendbar, wie z.B.
- Leber, mit folgenden Organfunktionszellen: Leberstammzellen, Hepatozyten, Kupfersche Sternzellen
- Schilddrüse: mit Epithelzellen und parafollikuläre Zellen als Funktionszellen
- Knorpel mit Chondrozyten
- Knochen mit Osteoblasten und Osteoklasten als Funktionszellen.

D.h., es wird die über längere Zeit verläßliche vermehrende oder wenigstens weitgehend erhaltende Kultivierung aller solcher Organfunktionszellen angestrebt.

Das zu diesem Zweck entwickelte erfindungsgemäße Verfahren der eingangs genannten Art ist im wesentlichen dadurch gekennzeichnet, daß die Kultivierung in einem Wirbelschichtreaktor mit blasenfreier Begasung im Reaktor selbst oder im Rezyklierungskreis an mit Strukturprotein oder Strukturprotein-Hydrolysat beschichteten, mit extrazellulärem Matrixprotein vorbehandelten und mit einer stromalen Zellschicht bedeckten makroporösen Glasträgern von maximal 1500 µm Durchmesser erfolgt.

Weitere Besonderheiten der Erfindung ergeben sich aus den Patentansprüchen und der nachfolgenden Beschreibung.

Zwar wurde bereits vor etwa zehn Jahren (DE-OS 3410650) vorgeschlagen, Zellkulturtechnik im Wirbelschichtreaktor mit makroporösen Glasträgerkörpern durchzuführen, und es wurde darauf aufbauend bereits über die Durchführung solcher Zellkulturen mit blasenfreier Begasung berichtet -siehe z.B. D. Looby, J. B. Griffiths: "Animal cell technology"; S. 336-344; Butterworth-Heinemann 1988 -, jedoch wurden dabei ausnahmslos Zellinien (d.h. ungegrenzt wuchernde Zellen ohne Organfunktion, z.B. für künstliche Proteinproduktion) verwendet.

Trotz der guten Bekanntheit dieser Wirbelschichttechnik haben sich die zahlreichen Bemühungen zur Erhaltung und Vermehrung von Organfunktionszellen bis in jüngste Zeit fruchtlos in andere Richtungen bewegt (s.o.).

Erst die erfindungsgemäße Technik der Besiedelung von großporigen Glasträgern (insbesondere von Glasträgern mit Makro- und Mikroporen), wie sie grundsätzlich aus der DE-OS 28 39 580 der Fa. Schott bekannt sind, die nur eine geringe Korngröße aufweisen, der eine geringe Fließgeschwindigkeit im Wirbelschichtreaktor entspricht, und deren Oberflächen mit einer dünnen Schicht von Strukturprotein (oder Hydrolysat wie insbesondere Gelatine) versehen und mit extrazellulärem Matrixprotein wie z.B. Fibronektin, Laminin, Heparinsulfat usw. behandelt und mit Stromazellen bedeckt sind, führt mit den zu vermehrenden Zellen zum gewünschten Erfolg der Erhaltung bzw. Vermehrung des Zellmaterials.

Dabei spielt die Großporigkeit und die geringe Korngröße der Träger eine wichtige Rolle: Insbesondere erstere fördert das Einnisten von Zellmaterial und dessen Kommunikation mit der Umgebung, so daß keine prägenden Akkumulationsbereiche für Hemmstoffe oder Abbauprodukte bestehen. Diese Hemmstoffe und Abbauprodukte stehen im Diffusionsgleichgewicht mit dem Medium, das zur Aufrechterhaltung des Fließgleichgewichtes, kontinuierlich in geregelter Konzentration bzw. kontrollierter, optimierter Konzentration die bewachsenen Trägerkörper anströmt.

Die Strömungsgeschwindigkeit des Wachstummediums ist - der geringen Größe der Träger (die im Fließzustand im Reaktor verbleiben und nicht ausgewaschen werden) angemessen - relativ gering, so daß keine erheblichen Scherkräfte auf den Bewuchs ausgeübt werden. Durch den kontinuierlichen Medienaustausch werden die ausdifferenzierten Zellen durch das strömende Medium ausgeschwemmt und aus dem Reaktor in das Produktgefäß ausgetragen. Das von den Stammzellen für die Selbstreplikation benötigte Gleichgewicht zwischen unreifen und ausgereiften Zellen wird so weitgehend erreicht.

Die als Produzent der Wachstums- oder Regulationsfakoren für die Erhaltung der Stammzellen wichtigen Stromazellen sind in Anbetracht der großen Porendurchmesser nicht von der Ernährung abgeschnitten und somit in der Lage, den Bedürfnissen der Stammzellen zu entsprechen.

Die Größe der Träger sollte in Bereich von 200-1500 µm liegen. Die Auswahl geeigneter Körngrößen der Glasträger wird zum einen durch die den Fließzustand der Wirbelschicht gewährleistende Strömungsgeschwin-digkeit im Reaktorrohr, die für eine Verwirbelung der Träger mit den gewünschten Stoffaustausch wie oben beschrieben sorgen soll, aber nicht so stark sein darf, daß das sessile Verhalten der Organfunktionszellen im Trägerhohlkörper gestört würde.

Allgemein können sich die Fließbedingungen physiologischen Größenordnungen annähern, wobei für die Zellen im Träger keine minder versorgten Bereiche auftreten sollten, welche bei größeren Trägern eher zu befürchten sind.

Zum anderen ist die Herstellbarkeit und "mechanische Festigkeit" der großporigen Träger zu berücksichtigen, deren Poren möglichst geräumige Besiedlungsnischen für die Zellpopulation bieten sollen und somit kaum kleiner als 10-20 µm sein dürfen, da Säugerzellen größer als 10 µm sind. Eine möglichst hohe Porosität von mindestens 40% und große offene Poren bis über 100 µm werden angestrebt.

Als besonders nützlich und verfügbar haben sich z.Zt. Träger mit Korngrößen. von 400-700 µm und Porengrößen um 50 µm erwiesen, deren Dichte im Bereich von 1,1 bis 1,8 kg/l (gemessen mit wassergefühlten Poren) liegt. Die erfindungsgemäß angestrebten Eigenschaften wären jedoch auch mit etwas größeren Trägerkörpern (wie z.B. bis 1500µm) erziehlbar, wobei im Falle besonders großer Trägerkörper eine gewisse Besiedlungssperre, der für den Stoffaustausch durch "versiegelnde" Zellschichten nicht ohne weiteres zugänglichen "inneren" Trägerbereichen, vorliegen könnte.

Der Auftrag dünner Beläge von Strukturprotein-(hydrolysat) auf Glas wirkt in Verbindung mit daran gebundenen Matrixproteinen, wie z.B. Fibronektin, als Haftvermittler zwischen Glas und Zellen.

Die Beschichtung mit z.B. Gelatine wird insbesondere durch Behandlung von hochgereinigtem Glas mit wasserfreier Gelatinelösung erreicht.

Ein Matrixproteinbelag wie insbesondere Fibronektinbelag kann darauf Behandlung mit serumhaltigem Medium erziehlt werden. Auch können die Matrixproteine in reiner Form zu den beschichteten Trägern für die Matrixproteinbelagbildung zugegeben werden.

Für die Anlagerung vor. stromalen Zellen auf der so vorbereiteten Oberfläche wird insbesondere eine Vorkultur mit Stomazellen mit möglichst geringen Strömungsgeschwindigkeiten durchgeführt, wobei möglichst eine Osmolarität von 300 mOsml eingestellt und die Medienzudosierung für 3-5 h unterbrochen werden sollte.

Die stromale Zellschicht auf den Trägern bietet den Organfunktionszellen die benötigte Anheftungsoberfläche. Für die Inokulierung der Organfunktionszellen werden die Zellen in frisches Medium aufgenommen und bilden so eine Suspension die in den Reaktor gegeben wird. Innerhalb von 2 h heften sich die Organfunktionszellen auf die Träger an und können so über einen sehr langen Zeitraum in aktiver Form aufrecht gehalten werden.

Es besteht so die Möglichkeit, ins Medium entlassene Produkte, wie Zellen, Zellgemische oder auch Proteine daraus kontinuierlich oder diskontinuierlich zu ernten oder die Organfunktionszellen durch Ausschleusung der bewachsenen Träger und ablösen der Zellen zu gewinnen.

Von stromalen Zellen abgegebene Wachstums- und Regulatorfakoren lassen sich analog ohne Besiedlung mit Organfunktionszellen gewinnen.

Träger, die mit Strukturproteinen oder zusätzlich mit Matrixproteinen beschichtet oder auch bereits mit stromalen Zellen besiedelt sind, können steril bevorratet und für Test-, Untersuchung- oder Produktionszwecken abgegeben werden.

Nachfolgend wird die Erfindung an Hand von Beispielen unter Bezugnahme auf die angefügten Zeichnungen beschrieben; es zeigen schematisch
- Fig. 1: eine besonders zweckmäßige Ausführungsart eines Wirbelschichtreaktors zur Durchführung des Verfahrens;
- Fig. 2: Wachstumskurve der Zelldichte und der entsprechenden Kapazität in Abhängigkeit von der mit Zeit von peripheren Stammzellen immobilisiert auf primären Stromazellen.
- Fig. 3: Wachstumskurve der Zelldichte entsprechenden Kapazität und der Verweilzeit gegen die Zeit. Kultiviert wurde aufgereinigtes Knochenmark mit Stromazellen.
- Fig. 4: Kurvenbild für die prozentuale Verteilung der Zelltypen Granulozyten, Monozyten, Leukozyten und Stromazellen) und die Zelldichte gegen die Zeit.
- Fig. 5: ein Trägerprobeentnahmesystem für die Anordnung nach Fig. 1., und
- Fig 6: einen Knochenquerschitt und einen bewachsenen makroporösen Träger, zum Vergleich.

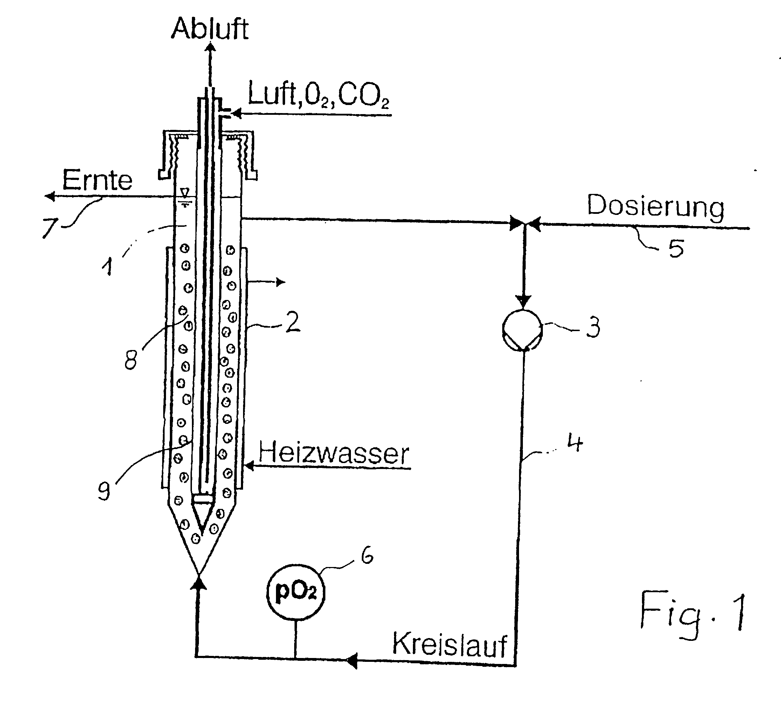

Figur 1 zeigt den Reaktor 1 mit Heizmantel 2 und mit einem von einer peristaltischen Umlaufpumpe 3 angetriebenem Rezyklierungskreis 4 für das flüssige Medium, das (schematisch mit 5 angedeutet) durch entsprechende Dosierstrategie auf optimalen Wachstums-/ Erhaltungsbedingungen gehalten wird. Durch 6 wird die Überwachung des Mediums bezüglich unterschiedlicher Parameter, insbesondere mit einer Sauerstoffsonde, angedeutet. Bei 7 wird dem Reaktor Medium (z.B. durch einen Überlaufanschluß entnomen. Zudosierung und Entnahme können auch im Kreislauf oder im Reaktor integriert sein.

Die Wirbelschicht 8 mit zellbewachsenen Trägern wird durch die unten in den Reaktor 1 einlaufende Rezyklierungsströmung im Fließzustand gehalten: Die Trägerkörper werden durch ihr höheres spezifisches Gewicht innerhalb der Reaktorröhre festgehalten und somit am Austragen am oberen Reaktorende in den Kreislauf gehindert. Innerhalb der zylindrischen Röhre, die das Wirbelbett enthält, ist koaxial ein Silikonmembranschlauch angebracht, über den der Sauerstoff für die Zellen eingetragen wird und der im wesentlichen, wie in dem DE-GM 9413576 beschrieben, ausgebildet ist. Die Silikonmembran kann aber auch in den Rezyklierungskreislauf integriert sein oder - bei größeren Reaktoren - in Mehrzahl vorgesehen werden.

Durch die Membrandiffusionsbegasung werden die Zellen keinen Scherkräften durch platzende Gasblasen ausgesetzt und es entstehen keine Probleme durch sonst not-wendige Antischaumzusätze. Gleichzeitig wird der pH-Wert über die Kohlendioxidkonzentration der Gasphase eingestellt.

Der Reaktor ist am unteren Ende konisch verjüngt und dem Durchmesser der Umlaufschläuche (kleiner 7,5 mm) angepaßt. Das Reaktorrohr hat z.B. selbst einen Durchmesser von ca. 10-30 mm bei einem Öffnungswinkel von ca. 25°.

Die unreiferen hämapoetischen Zellen sind vollständig oder mehrheitlich an die Oberflächen und in den Poren der porösen Trägerkörper angeheftet. Die reiferen Zellen lösen sich von den Trägern ab und gelangen über den Umlauf ins Produktgefäß. Im Beispiel wurden cffenporige Trägerkörper aus Borosilikatglas (Siran® der Fa. Schott, Mainz) mit einer Dichte von ca. 1,6 kg/l benutzt. Ihr Hohlraumanteil (Porosität) lag bei ca. 50%, ihr Durchmesser bei 400-700µm und die visuell ermittelte mittlere Porengröße um etwa 50µm.

Die Zelldichten, die insgesamt erreicht wurden, lagen je nach Zelltyp zwischen 15-200 Millionen Zellen pro ml Wirbelkörperschüttung.

Der Reaktor wurde kontinuierlich betrieben, die Zudcsierung erfolgte über eine Zuleitung im Kreislauf, die Ernte über einen Überlaufanschluß im Reaktor.

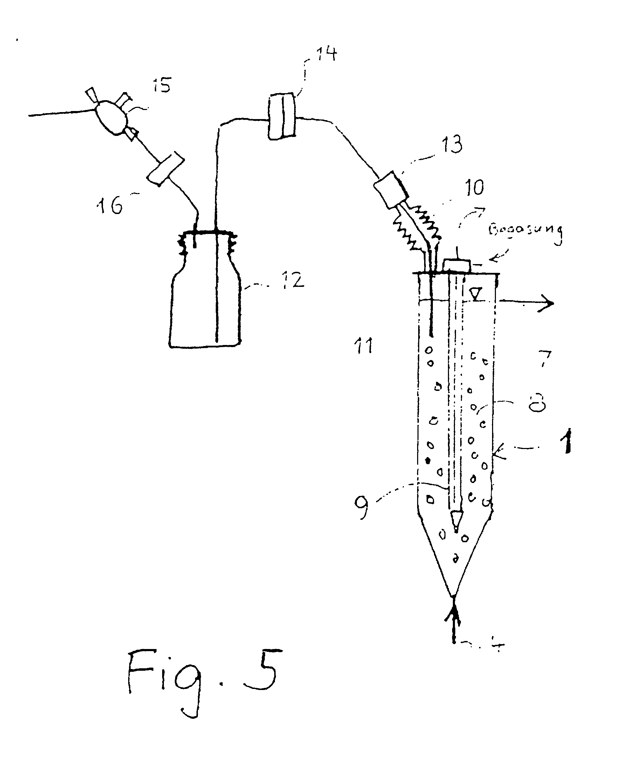

Zur Entnahme von Trägerkörpern kann der Reaktordeckel geöffnet werden. Ein Trägerprobeentnahmesystem kann in den Deckel integriert sein (s. Fig 5). Das Trägerprobeentnahmesystem besteht aus einem Faltenbalg (variabler Länge von 12-23 cm), in dem sich ein Teflonschlauch befindet. Im ausgefahrenen (gedehnten) Zustand des Faltenbalges befindet sich der Teflonschlauch oberhalb des Reaktormediums, im Kopf der Reaktorröhre. Somit behindert der Schlauch nicht die Strömungseigenschaften des Reaktors. Im eingefahrenen Zustand befindet sich der Schlauch im Wirbelbett. Die Trägerentnahme erfolgt durch einen Unterdruck an dem Schlauchende. Die Träger werden in eine Flasche am Ende des Schlauches gesogen, die dann steril gewechselt wird. Medium und Trägerreste im Schlauch können durch Überdruck zurück in den Reaktor befördert werden. Somit kann eine Trägerprobe steril aus dem Reaktor entnommcn werden.

Kenndaten des Reaktors:
- Schüttvolumen der Trägerkörper pro Gesamtvolumen des Reaktor (einschließlich Umlauf) = 0,25 (Werte zwischen 0,2 und 0,6 sind brauchbar)
- Schüttvolumen = 50ml
- Reaktorvolumen = 200 ml
- Membranfläche pro Schüttvolumen WK = 10 cm²/ml
- Inokulum: mindestens 1*10⁵ Zellen pro ml Träger

### Behandlung der Träger:

Für das erfindungsgemäße Verfahren wurden die Träger zunächst folgendermaßen vorbereitet und beschichtet:

### Reinigung der Glasträger

1 Liter Glasträger wurden in 1,5 Liter 2,5M Salzsäure 12h bei 100°C erwärmt. Danach wurden sie 2 mal mit 10 Liter hochreinem Wasser gewaschen.

Weiter wurden die Träger mit 1,5 Liter 2,5%iger Salpetersäure 12h bei 100°C erwärmt. Danach wurde sie 2 mal mit 10 Liter hochreinem Wasser säurefrei gewaschen.

### Trocknung

Die Träger wurden 6-8 h bei 220°C im Trockenschrank getrocknet.

### Beschichtung mit Gelatine

1 Liter trockene Träger wurden mit 1,2 Liter Dimethylsulfoxid (DMSO) überschichtet und dann mit in 450 ml DMSO gelöster Gelatine versetzt (15g/l). Dann wurden die Träger 3 Tage bei 50°C geschüttelt.

Die Träger wurden dann zur Entfernung von DMSO und die überschüssiger Gelatine 10 mal mit 10 l hochreinem, 60°C warmen Wasser gewaschen.

Die Träger wurden im Autoklaven sterilisiert und waren so für die Kultivierung im Wirbelschichtreaktor einseczbar.

Ergebnis ist sehr dünne Schicht aus Gelatine, die den Träger überzieht, ohne die Poren zu verstopfen.

Als Apparatur diente ein 200ml Wirbelschichtreaktor gemäß Fig. 1, aber mit externer blasenfreier Begasung (Membran-Begasung) im Rezyklierungskreis mittels Silikonschlauch (4m => 0,075m² Oberfläche), mit einer auf Kapazitätsmessung basierenden Biomassensonde zur Überwachung der Biomassendichte und mit Sonden zur Kontrolle des pH-Wertes und des Sauerstoffsättigungswertes.

Die Sonden wurden geeicht und der gesamte Reaktor autoklaviert. Der abgekühlte Reaktor wurde mit Medium und mit der. wie oben beschrieben gelatinebeschichteten Glasträgern (50 ml Schüttvolumen) steril befüllt und einem 3-tägigen Steriltest unterzogen.

Das Medium bestand aus käuflichem α-Medium der Firma GIBCO, dem 10% fötales Kälberserum (Firma Boehringer Mannheim), 2g/l Glucose, 4,3g/l HEPES und 2,2g/l NaH-CO₃ zugesetzt wurden. Durch diese Behandlung bindet das Fibronektin des Kälberserums an die Gelatine. Anschließend wurde der sterile Reaktor nochmals mit Medium gespült, um die Abwesenheit von Zerfallsprodukten des Mediums sicherzustellen.

### Beispiel 1

Der wie oben beschrieben vorbereitete Reaktor wurde mit Knochenmarkstromafibroblasten beimpft. Als Inokulum dienten 3,6*10⁷ Zellen entsprechend 7,2*10⁵ Zellen pro ml Träger.

Am Tag 5 wurden 4*10⁶ periphere Stammzellen (entsprechend 8*10⁴ Zellen pro ml Träger) nachinokuliert.

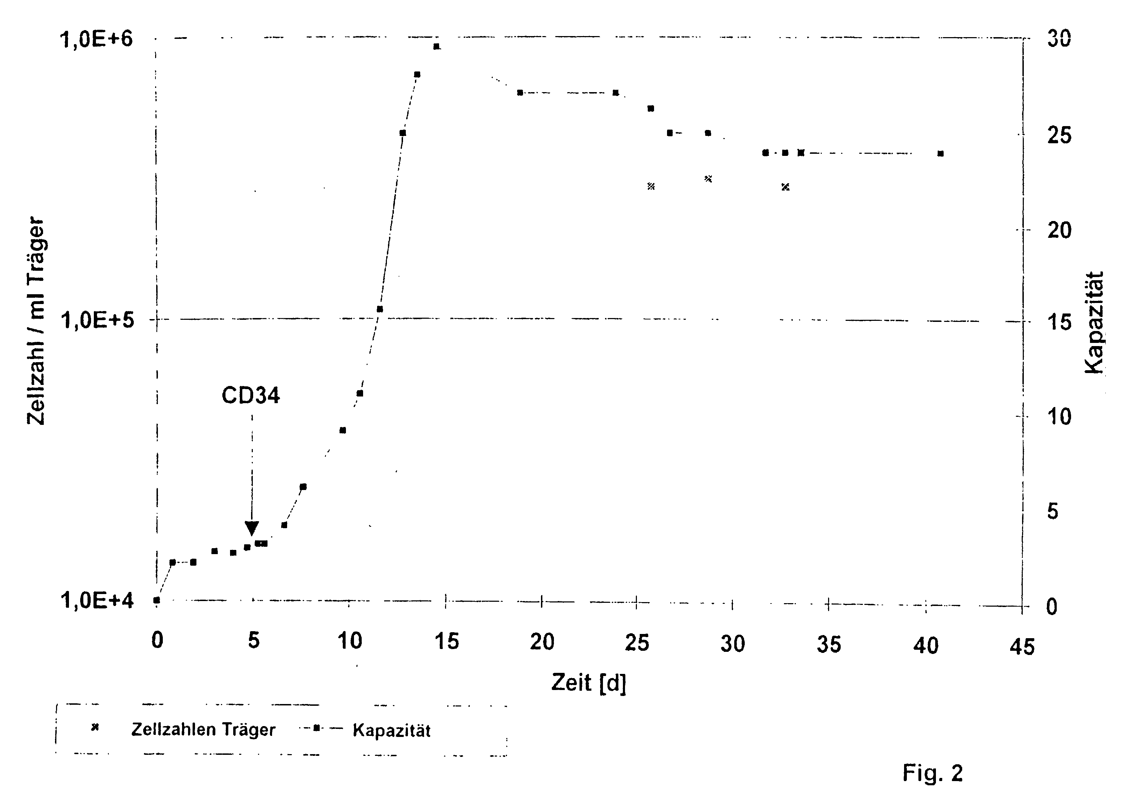

Pro Tag wurden dem Reaktor 1-2 Mediumproben, zur Bestimmung der Glukose-, Laktat- und Aminosäurenkonzentrationen im Reaktor entnommen. Alle 2-3 Tage wurden Trägerproben für eine Kernzellzählung mittels Kristallviolett-Citrat-Test entnommen, um die Zellzahlen auf den Trägern zu bestimmen. Die Ergebnisse wurden mit den entsprechenden Bestimmungen mittels der Biomassensondekapazitätsmessung verglichen. Das Ergebnisse sind in den beigefügten Fig. 2 dargestellt.

Man sieht den praktisch konstanten Verlauf der Fibroblastenkonzentration auf den Trägern, sowie die nach Zugabe von peripheren Stammzellen (CD 34) stattfindende exponentielle Zunahme der Zellzahlen und der Kapazität.

Dabei zeigt sich eine einigermaßen zufriedenstellende Übereinstimmung zwischen den in unterschiedlicher Weise erhaltenen Ergebnissen der Zellzahlbestimmung.

Die exponentielle Zunahme der Zellzahlen geht nach 14-15 Tagen in einen langsamen Abfall über, der in einem horizontalen Ast endet, der einer Gleichgewichtseinstellung mit einer Endzelldichte on 5-8*10⁵ Zellen pro ml Träger und einem Kapazitätswert von ca 25pF zu entsprechen scheint. Ein Vergleich der Impfmenge mit dem Gleichgewichtswert läßt eine Verzehnfachung der Zelldichte erkennen.

Parallel zur Zelldichte wurde die Glukosezudosierung und die Glukoseverbrauchsrate über die Versuchszeit hinweg bestimmt: Dabei zeigt sich nach anfänglich geringer Zudosierungs- und Verbrauchsrate in der Anwachsphase der Stromafibroblasten, ab der Nachimpfung mit Stammzellen (Tag 5), eine Zunahme des Glukoseverbrauchs bei verdoppeltem Glukosezusatz. Auch bezüglich der Glukoseverbrauchsrate wird nach 15 Tagen ein etwa konstanter Wert erreicht.

Somit konnte gezeigt werden, daß die Stromazellen auf den Trägern immobilisiert werden konnten und keine starke Vermehrung dieser Zellen auftritt. Damit liegt eine stabile wachstumsunterstützende Zellschicht vor an der sich die Stammzellen anheften. Das Anheften und die Vermehrung der Stammzellen unter Ausdifferenzierung konnte an Hand der exponentiellen Zunahme der Zellzahl gezeigt werden. Die einzelnen Zelltypen würden nicht bestimmt.

### Beispiel 2

Es wurden vorbereitete Träger wie in Beispiel 1 in dem beschriebenen Reaktcr unter den genannten Bedingungen verwendet. In Beispiel 2 wurden die Fibroblasten aus Zeitmangel nur 12 h vorkultiviert.

Auf die so vorbehandelten Träger wurde aufgereinigtes Knochenmark gegeben, aus dem die Erythrozyten und Teile der Lymphocyten entfernt worden waren.

Die Wirbelschichtkultur erfolgte wie in Beispiel 1.

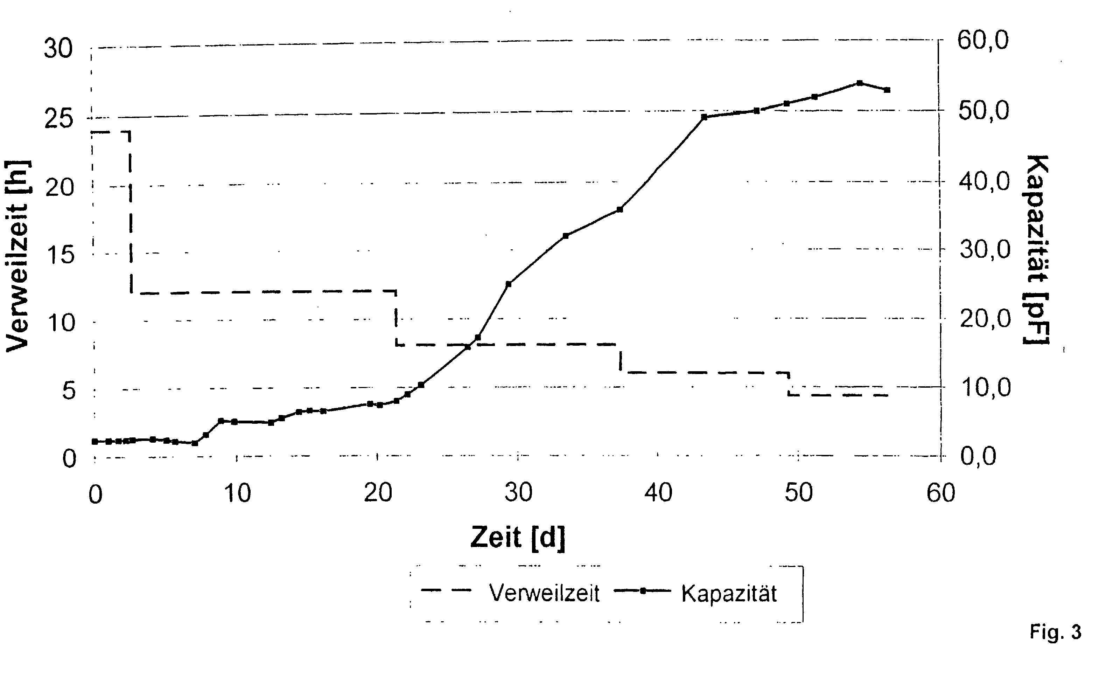

In Fig. 3 sind die Kapazitätsmessung mit der Biomassensonde und die Verweilzeit über die Zeit aufgetragen. Wie zu erkennen ist, nimmt die Kapazität und damit die Zellzahl zunächst recht langsam zu und erreicht nach 40 Tagen eine Maximalwert von über 50 pF, was einer Zellzahl von über 1*10⁴ Zellen pro ml Träger entspricht. An Hand der Kapazität ist das exponentielle Wachstum der hämapoetischen Zellen zu erkennen. Die Verweilzeit des Mediums nimmt bei der Zunahme der Zelldichte ab und erreicht einen Endwert von 4,6h.

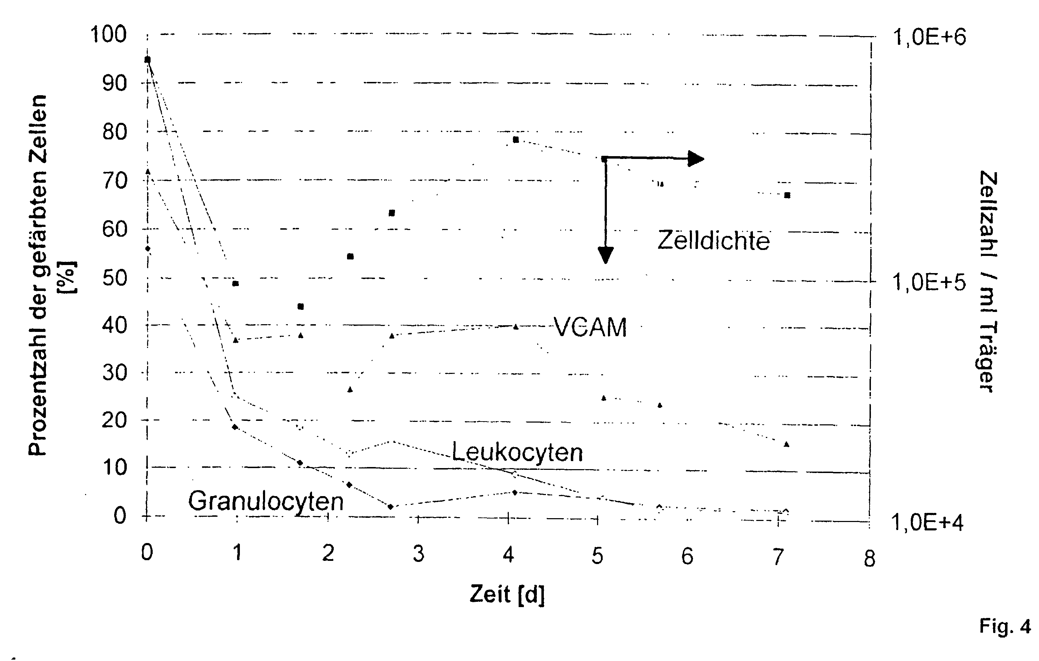

Weiterhin konnten, wie in Figur 4 zu erkennen ist, die einzelnen Zelltypen an Hand von Flow Cytomer Messung mittels CD-Markern bestimmt werden. Dabei zeigte sich, daß die bei der Bestimmung angefärben spezifischen Rezeptoren nicht lange exprimiert werden und nur die ersten Tage für die Zelltypisierung verwendet werden können.

Durch zellphysiologische Untersuchungen könnten aber die Zelltypen bestimmt werden.

Einen Tag nach dem Nachinokulieren des Knochenmarks wurde eine Trägerprobe aus dem Reaktor entnommen und die darauf befindlichen Zellen auf ihre Typen untersucht. Dabei zeigt sich das Prozentual zum Inokulum die frühen Progenitorzellen (CD34+ und CD38- = andifferenzierte Stammzellen) des Knochenmarks zu 40% und die späten zu 50% (CD38+) an die Träger anheften.

Stammzellen können mit dem Flow Zytometer nicht nachgewiesen werden, bilden aber einen sehr geringen Teil der Progenitorzellen.

Im Gegensatz dazu immobilisieren aber die mehr ausdifferenzierten Zellen nur in einem sehr geringen Maße, z.B. alle Leukozyten zu 8%, die granulozytären Zellen und die monozytären Zellen zu 15%. Zu den Leukozyten gehören alle andifferenzierten weißen Blutzellen, somit auch ein die Granulozyten und Monozyten.

Die vorkultivierten Träger eignen sich somit auch zur Aufreinigung der Progenitorzellen aus Knochenmark oder Blut. Weiterhin beweisen diese Ergebnisse auch, daß sich hauptsächlich frühe hämapoetische Zellen an die Träger anheften und nur zu einem geringen Teil die reifen Blutzellen die eine Selbstreplikation der Stammzellen behindern würden.

In Tabelle 1 sind die Ergebnisse zusammengefaßt.

**Tabelle 1:**

| Prozentsatz der immobilisierten Zellen bezogen auf Inokulumsmenge des Knochenmarks | | | |
|---|---|---|---|
| T-Helfer-Zellen | 0% | T-Suppressorzellen | 8% |
| Granulozyten | 15% | Monozyten | 15% |
| frühe Erythrozyten | 8% | Leukozyten | 8% |
| frühe Progenitorz. | 40% | spätere Progenitorz. | 50% |

Kultivierungen von peripheren Stammzellen und von angereichertem Knochenmark, ohne das die Träger mit Stromazellen beschichtete waren, führten nicht zum Er folg, da sich die hämapoetischen Zellen nicht an die Träger anhefteten.

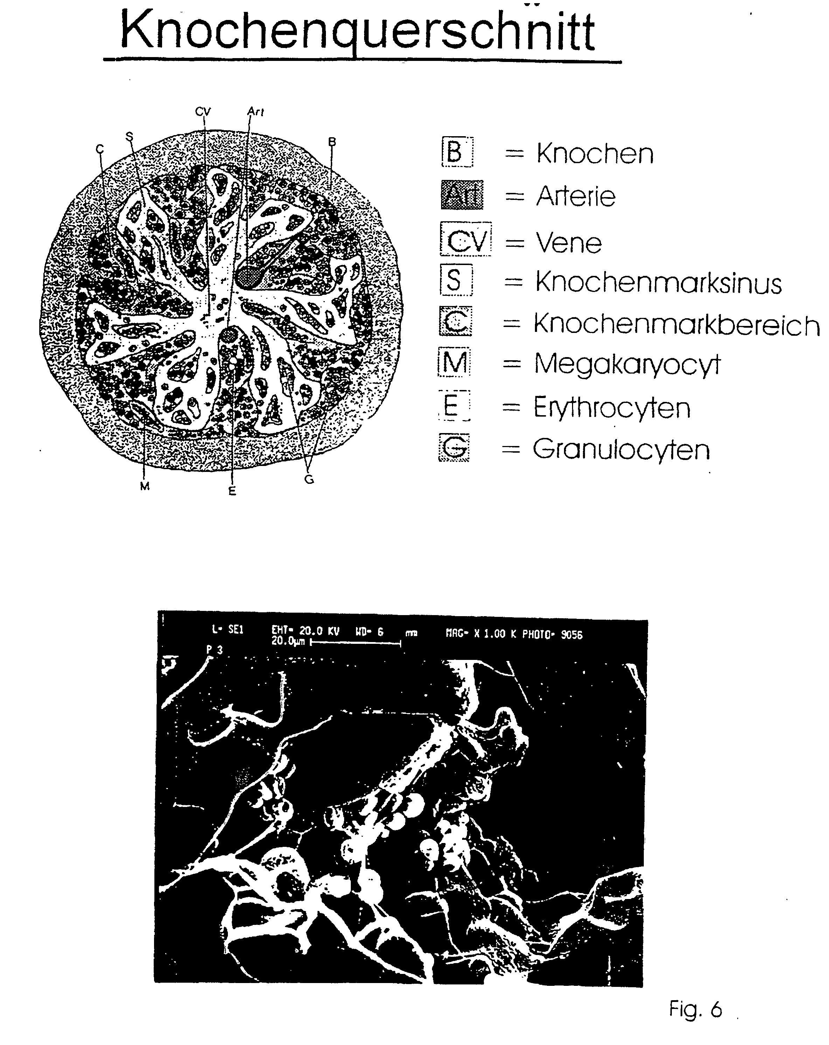

In Fig. 6 ist ein Querschitt eines Knochens mit dem darin enthaltener. Knochenmark und die Struktur der Träger im bewachsenen Zustand verglichen. Dabei zeigt sich eine gute Übereinstimmung der Zellstruktur auf dem hochporösen Träger mit dem Knochen.

### Beispiel 3

In einem weiteren Versuch wurden nur primäre Stromazellen in dem in Fig. 1 beschriebenen Wirbelschichtreaktor kultiviert, um die wachstumsunterstützende Zellschicht besser charakterisieren zu können. Dabei zeigt sich ein sehr langsames Wachstum der Stromafibroblasten mit einer Verdopplundszeit von ungefähr 130 h. Damit konnte eire maximale Zelldichte von 1 Millionen Zellen pro ml Trägern erreicht werden, die über 1000 h konstant aufrecht gehalten werden konnte.

Folgende Zytokinkonzentrationen konnten im stabilen Zustand ermittelt werden.

**Tabelle 2:**

| Medienzytokinkonzentrationen im stationären Zustand der Fermentation | | | |
|---|---|---|---|
| PDGF | 10 pg/ml | TGF | 500 pg/ml |
| G-CSF | >2000 pg/ml | GM-CSF | 1700 pg/ml |
| M-CSF | 7000 pg/ml | MIP 1a | 135 pg/ml |
| IL-6 | >3000 pg/ml | IL-1l | >3033 pg/ml |
| bFGF | >40 pg/ml | LIF | 1700 pg/ml |

Die vorstehend mitgeteilten Ergebnisse erlauben die Feststellung, daß erfindungsgemäß Stromafibroblastenzellen auf den Trägern kultiviert werden können und in dieser Form als Cytokinlieferanten wirken. Dieser Fibroblastenbewuchs ermöglicht das Anheften, das Wachstum und die Differenzierung von pluripotenten Stammzellen.

Um die Stammzellen gewinnen zu können, werden die bewachsenen Träger mittels des Trägerprobeentnahmesystem entnommen, zweimal mit Pufferlösung gewaschen und dann durch ei-ne 10%ige Trypsinlösung von den Trägern gelöst. Die dadurch gewonnen Zellen sind lebend und können für jegliche Anwendungen benutzt werden.

Diese Versuche dürfen als Hinweis auf die Überlegenheit ces erfindungsgemäßen Verfahrens bezüglich der Erhaitung bzw. zur Vermehrung führende Kultivierung von pluripotenten Stammzellen gewertet werden. Trypsin nur kurz angewendet (<20 min)führt zu keiner Zellschädigung.

Die am Beispiel Knochenmark gezeigte Nützlichkeit kann selbstverständlich für andere organähnliche Mischpopulationen ausgenutzt werden.

So könnten z.B. Leberfunktionszellen (Hepatezyten) auf mit Stromazellen beschichteten Trägern immobilisiert und so die Leberfunktion eines Patienten ersetzt werden. Weiterhin könnte man den Abbau von Pharmazeutika in der Leber simulieren.

Auch könnte die Wirkung von Pharmazeutika oder Hormonen auf Schilddrüsensimulate in Wirbelschichtreaktoren untersucht werden. Als weitere Anwendungsmöglichkeit wäre die Immobilisierung von Osteoblasten auf mit Stromazellen beschichtete Träger denkbar, deren Wachstum stimmuliert und damit in großen Zellmengen produziert würden.

## Patentansprüche

1. Verfahren zur Kultivierung von menschlichen bzw. tierischen Organfunktionszellen, bei dem auf porösen Mikroträgern angesiedelte Zellen kontinuierlich mit Wachstumsmedium versorgt werden,
**dadurch gekennzeichnet, daß**
die Kultivierung in einem Wirbelschichtreaktor mit blasenfreier Begasung im Reaktor selbst oder im Rezyklierungskreis an mit Strukturproteinen oder mit Strukturproteinhydrolysat beschichteten, mit extrazellulären Matrixproteinen vorbehandelten und mit einer stromalen Zellschicht bedeckten makroporösen Glasträgern von maximal 1500 µm Durchmesser erfolgt.

2. Verfahren nach Anspruch 1,
**dadurch gekennzeichnet, daß**
mit Gelatine beschichtete und mit Fibronektin vorbehandelte Träger in einer Vorkultur mit Stromazellen angereichert werden, bevor die Beimpfung mit Organfunktionszellen erfolgt.

3. Verfahren nach Anspruch 1 oder 2,
**dadurch gekennzeichnet, daß**
die Vorkultur mit Knochenmark-Stromazellen durchgeführt wird und als Organfunktionszellen periphere Stammzellen oder Knochenmark eingesetzt werden.

4. Verfahren nach einem der vorangehenden Ansprüche,
**dadurch gekennzeichnet, daß**
die Vorbehandlung mit Fibronektin durch die Behandlung der Träger im Reaktor mit Kälberserum enthaltendem Medium erfolgt.

5. Verfahren nach einem der vorangehenden Ansprüche,
**dadurch gekennzeichnet, daß**
mit Glasträgern von 400 - 700 µm und einer Trägerporosität um 50 % und einer Porengröße von 30 - 120 µm gearbeitet wird.

6. Verfahren nach einem der vorangehenden Ansprüche,
**dadurch gekennzeichnet, daß**
der prozentuale Anteil des Zwischenkornvolumens zum gesamten Wirbelschichtvolumen 50 - 75 % beträgt (Wirbelschichtporosität) und mit einem Trägeranteil in der Wirbelschicht von 20 - 60 % Träger-Schüttvolumen bezogen auf das Arbeitsvolumen des Reaktors einschließlich des Rezyklierungskreises gearbeitet wird.

7. Verfahren nach einem der vorangehenden Ansprüche,
**dadurch gekennzeichnet, daß**
mit kontinuierlicher und geregelter Mediumzufuhr gearbeitet wird, wobei die Verweilzeit unter 72 h, insbesondere 10 - 50 h betragen sollte.

8. Verfahren nach Anspruch 7,
**dadurch gekennzeichnet**,
das der Trägerbewuchs im Reaktor mit einer auf Kapazitätsmessung beruhenden Biomassesonde überwacht wird, deren Information als Regelgröße für die Zellzahl und damit für die Medienzufuhr dient.

9. Verfahren nach einem der vorangehenden Ansprüche,
**dadurch gekennzeichnet, daß**
die Kultivierung zur Erzeugung von Organsimulaten durchgeführt wird.

10. Verfahren nach einem der vorangehenden Ansprüche,
**dadurch gekennzeichnet, daß**
Leber- bzw. Knochenmarkfunktionszellen insbesondere in Langzeitkulturen erzeugt werden.

11. Verfahren nach einem der vorangehenden Ansprüche,
**dadurch gekennzeichnet, daß**
reife Blutzellen durch ein immobilisiertes hämapoetisches System erzeugt werden.

## Claims

1. Method of cultivating functional cells of human or animal organs, in which cells colonized on porous microcarriers are continuously supplied with growth medium,
**characterized in that**,
the cultivation is carried out in a fluidized bed reactor with bubble-free aeration in the reactor itself or in the recycling circuit, using macroporous glass carriers having a maximum diameter of 1500 µm, which have been coated with structural proteins or with structural protein hydrolysate, pre-treated with extracellular matrix proteins and covered with a layer of stromal cells.

2. Method according to Claim 1,
**characterized in that**
carriers coated with gelatin and pre-treated with fibronectin are enriched in a preculture with stroma cells, before inoculation with organ functional cells.

3. Method according to Claim 1 or 2,
**characterized in that**
the preculture is carried out with bone-marrow stroma cells, and peripheral stem cells or bone marrow are used as organ functional cells.

4. Method according to one of the preceding claims,
**characterized in that**
the pre-treatment with fibronectin is effected by treating the carriers in the reactor with a medium containing calf serum.

5. Method according to one of the preceding claims,
**characterized in that**
glass carriers of 400 - 700 µm and a carrier porosity of around 50% and a pore size of 30 - 120 µm are used.

6. Method according to one of the preceding claims,
**characterized in that**
the proportion of void volume to total fluidized bed volume is 50 - 75% (fluidized bed porosity), and there is a proportion of carriers in the fluidized bed of 20 - 60% bulk volume of carriers relative to the working volume of the reactor including the recycling circuit.

7. Method according to one of the preceding claims,
**characterized in that**
the supply of medium is continuous and controlled, and the sojourn time should be less than 72 h, more specifically 10-50 h.

8. Method according to Claim 7,
**characterized in that**
the carrier growth in the reactor is monitored by a biomass probe based on measurement of capacitance, the probe information serving as control variable for the cell count and thus for the supply of media.

9. Method according to one of the preceding claims,
**characterized in that**
the cultivation is carried out to produce organ simulates.

10. Method according to one of the preceding claims,
**characterized in that**
liver or bone-marrow functional cells are produced especially in long-term cultures.

11. Method according to one of the preceding claims,
**characterized in that**
mature blood cells are produced by an immobilized haemapoietic system.

## Revendications

1. Procédé de culture de cellules fonctionnelles d'organes humaines ou animales dans lequel les cellules installées sur des microsupports poreux sont alimentées en continu avec le milieu de croissance, **caractérisé en ce que** la culture s'effectue dans un réacteur à lit fluidisé avec un gazage sans bulles dans le réacteur lui-même ou dans le circuit de recyclage sur des supports en verre macroporeux revêtus de protéines structurelles ou d'hydrolysat de protéines structurelles, prétraités avec des protéines matricielles extracellulaires et recouverts d'une couche de cellules stromales, d'un diamètre maximal de 1500 µm.

2. Procédé selon la revendication 1, **caractérisé en ce que** les supports revêtus de gélatine et prétraités avec de la fibronectine sont enrichis dans une préculture avec des cellules stromales, avant l'inoculation avec les cellules fonctionnelles d'organes.

3. Procédé selon la revendication 1 ou 2, **caractérisé en ce que** la préculture est réalisée avec des cellules stromales de moelle osseuse et que l'on met en oeuvre comme cellules fonctionnelles d'organe des cellules souches périphériques ou de la moelle osseuse.

4. Procédé selon l'une des revendications précédentes, **caractérisé en ce que** le prétraitement avec la fibronectine s'effectue par le biais du traitement des supports dans le réacteur avec un milieu contenant du sérum de veau.

5. Procédé selon l'une quelconque des revendications précédentes, **caractérisé en ce que** l'on travaille avec des supports en verre de 400 à 700 µm et une porosité des supports de 50 % et une taille des pores de 30 à 120 µm.

6. Procédé selon l'une quelconque des revendications précédentes, **caractérisé en ce que** le pourcentage du volume des grains intermédiaire sur le volume total du lit fluidisé est de 50 à 75 % (porosité du lit fluidisé) et que l'on travaille avec une fraction de supports dans le lit fluidisé de 20 à 60 % en volume de vrac de supports sur le volume utile du réacteur, circuit de recyclage compris.

7. Procédé selon l'une quelconque des revendications précédentes, **caractérisé en ce que** l'on travaille avec un apport de milieu en continu et réglé, le temps de résidence devant être inférieur à 72 h, en particulier se situer entre 10 et 50 h.

8. Procédé selon la revendication 7, **caractérisé en ce que** la culture du support dans le réacteur est contrôlée avec une sonde de biomasse reposant sur la mesure de la capacité, dont les informations servent de valeur de réglage pour le nombre de cellules et donc pour l'apport du milieu.

9. Procédé selon l'une quelconque des revendications précédentes, **caractérisé en ce que** la culture est réalisée pour produire des ersatz d'organe.

10. Procédé selon l'une quelconque des revendications précédentes, **caractérisé en ce que** les cellules fonctionnelles du foie ou de la moelle osseuse sont produites en particulier dans des cultures de longue durée.

11. Procédé selon l'une quelconque des revendications précédentes, **caractérisé en ce que** les cellules sanguines matures sont produites par le biais d'un système hématopoïétique immobilisé.
